# EUROPÄISCHE PATENTSCHRIFT

(11) **EP 4 445 928 B1**
(45) Veröffentlichungstag und Bekanntmachung des Hinweises auf die Patenterteilung: **14.01.2026**
(21) Anmeldenummer: 23168004.2
(22) Anmeldetag: 14.04.2023
(51) Int. Cl.: A61M 5/24, A61M 5/50, A61M 5/31

(54) **SPRITZE MIT ORIGINALITÄTSVERSCHLUSS**
SYRINGE WITH TAMPER-EVIDENT CLOSURE
SERINGUE AVEC FERMETURE ANTI-REUTILISATION

(43) Veröffentlichungstag der Anmeldung: 16.10.2024
(73) Patentinhaber: Transcoject GmbH, 24539 Neumünster (DE)
(72) Erfinder: Heinz, Jochen, 24220 Flintbek (DE)
(74) Vertreter: Patentanwälte Hemmer Lindfeld Frese Partnerschaft mbB

(56) Entgegenhaltungen:
- DE-A1- 102018 111 449
- US-A1- 2004 116 858
- US-A1- 2015 045 744
- US-A1- 2015 246 185
- US-A1- 2016 001 015
- US-A1- 2016 151 584
- US-A1- 2022 362 475

## Beschreibung

Die Erfindung betrifft eine Spritze mit einem Spritzenzylinder und insbesondere eine vorgefüllte Spritze.

Bei vorgefüllten Spritzen ist es bekannt, den Luer-Anschluss des Spritzenzylinders mit einem Originalitätsverschluss zu verschließen. Aus EP 2 900 301 B1 ist ein entsprechender Verschluss bekannt, welcher über eine Sollbruchstelle zu öffnen ist. US 2004/0116858 A1 offenbart einen Originalitätsverschluss für eine Spritze, bei welcher ein Verschlusselement mit der Spritze verschweißt ist. Das Verschlusselement weist beabstandet zu der Schweißnaht eine vordefinierte Sollbruchstelle auf, an welcher das Verschlusselement geöffnet werden kann, um einen im Inneren gelegenen Luer-Anschluss zugänglich zu machen.

Es ist Aufgabe der Erfindung, eine Spritze, einen Spritzenzylinder mit einem Luer-Lock-Anschluss dahingehend weiterzuentwickeln, dass ein einfach anzubringender und zuverlässig und leicht zu öffnender Originalitätsverschluss bereitgestellt wird.

Diese Aufgabe wird gelöst durch eine Spitze mit den in Anspruch 1 angegebenen Merkmalen. Bevorzugte Ausführungsformen ergeben sich aus den Unteransprüchen, der nachfolgenden Beschreibung sowie den beigefügten Figuren.

Die erfindungsgemäße Spritze weist einen Spritzenzylinder auf, welcher beispielsweise aus Kunststoff gefertigt sein kann. Der Spritzenzylinder ist an einem ersten Axialende offen ausgebildet und weist an seinem entgegengesetzten zweiten Axialende, dem distalen Axialende, eine Stirnwand mit einem an dieser ausgebildeten Luer-Lock-Anschluss auf. Der Luer-Lock-Anschluss ist dabei bevorzugt mittig an der Stirnwand der zweiten Axialseite platziert. Der Luer-Konus des Luer-Lock-Anschlusses weist eine Öffnung zum Inneren des Spritzenzylinders auf. Ferner ist ein den Luer-Lock-Anschluss verschließender Originalitätsverschluss vorhanden, welcher mit dem Spritzenzylinder über zumindest eine Sollbruchstelle verbunden ist. Durch Brechen bzw. Lösen der Sollbruchstelle kann der Originalitätsverschluss geöffnet und vom Spritzenzylinder abgenommen werden.

Der Originalitätsverschluss weist einen ersten ringförmigen Abschnitt auf, der mit einem Außengewinde versehen ist. Dieses Außengewinde ist korrespondierend zu dem Innengewinde des Luer-Lock-Anschlusses ausgebildet, sodass es schraubend mit diesem in Eingriff treten kann. Wenn der Originalitätsverschluss an dem Spritzenzylinder befestigt ist und auf den Luer-Lock-Anschluss aufgesetzt ist, greift das Außengewinde an dem ersten ringförmigen Abschnitt in das Innengewinde am äußeren zylindrischen Abschnitt des Luer-Lock-Anschlusses ein. Das Innengewinde am Luer-Lock-Anschluss ist standardmäßig ausgebildet und dient nach Abnehmen des Originalitätsverschlusses der Befestigung eines korrespondierenden Luer-Lock-Verbinders.

Der Originalitätsverschluss weist darüber hinaus in einer Ausgestaltung der Erfindung einen zweiten ringförmigen Abschnitt auf, der den äußeren zylindrischen Abschnitt des Luer-Lock-Anschlusses umfänglich umgibt, vorzugsweise konzentrisch umgibt. Dabei ist der zweite ringförmige Abschnitt radial beabstandet von dem ersten ringförmigen Abschnitt, sodass ein ringförmiger Spalt zwischen den beiden ringförmigen Abschnitten gebildet ist, in welchen der äußere zylindrische Abschnitt des Luer-Lock-Anschlusses eingreift.

Die zumindest eine Sollbruchstelle ist zwischen dem ersten ringförmigen Abschnitt und dem Spritzenzylinder und/oder zwischen dem zweiten ringförmigen Abschnitt und dem Spritzenzylinder ausgebildet. Dazu ist bei einer ersten möglichen Ausgestaltung der erste ringförmige Abschnitt an einem ersten Axialende über die zumindest eine Sollbruchstelle mit dem Spritzenzylinder verbunden. Alternativ oder zusätzlich ist der zweite ringförmige Abschnitt an einem ersten Axialende über zumindest eine Sollbruchstelle mit dem Spritzenzylinder verbunden. Über diese Verbindung wird der Originalitätsverschluss an dem Spritzenzylinder so gehalten, dass er durch Zerbrechen oder Zerreißen der Sollbruchstelle oder Sollbruchstellen gelöst werden kann. Nach dem Lösen kann der Originalitätsverschluss durch Drehen in dem Gewinde abgenommen werden. Die Zerstörung der Sollbruchstelle bleibt dabei erkennbar, sodass sichtbar ist, ob sich der Originalitätsverschluss in einem unbeschädigten versiegelten Zustand befindet oder bereits einmal geöffnet worden ist.

Durch den Gewindeeingriff zwischen dem Originalitätsverschluss und dem am Luer-Lock-Anschluss vorhandenen Innengewinde wird der Originalitätsverschluss beim Öffnen besser geführt. Wenn der Originalitätsverschluss gedreht wird, wird die Sollbruchstelle durch die Drehbewegung geöffnet bzw. gebrochen. Über den Gewindeeingriff wird dabei gleichzeitig der Originalitätsverschluss in axialer Richtung von dem Spritzenzylinder wegbewegt. So wird eine sehr einfache Handhabung beim Abnehmen des Originalitätsverschlusses erreicht.

Der Originalitätsverschluss weist gemäß einer bevorzugten Ausführungsform der Erfindung ein an dem Luer-Konus des Luer-Lock-Anschlusses anliegendes elastisches Dichtelement auf. Das Dichtelement dichtet die Öffnung im Luer-Konus ab, solange der Originalitätsverschluss mit dem Spritzenzylinder verbunden ist. So wird ein sicherer Verschluss geschaffen, was insbesondere bei einem vorgefüllten Spritzenzylinder wichtig ist.

Das elastische Dichtelement ist vorzugsweise im Inneren des ersten ringförmigen Abschnittes angeordnet. So kann es an der Öffnung des Luer-Konus zur Anlage kommen bzw. den Luer-Konus durch Anlage am Luer-Konus dichtend verschließen.

Das Dichtelement ist bevorzugt aus einem Elastomer, beispielsweise Gummi gefertigt. Durch die Elastizität wird eine dichtende Anlage am Luer-Konus gewährleistet.

Das Dichtelement kann kraft- und/oder formschlüssig in dem ersten ringförmigen Abschnitt fixiert sein. So kann das Dichtelement nach dem Ausbilden der übrigen Struktur des Originalitätsverschlusses in diesen kraft- und/oder formschlüssig eingesetzt sein. Beispielsweise kann die übrige Struktur des Originalitätsverschlusses, welche insbesondere den ersten ringförmigen Abschnitt und ggf. den zweiten ringförmigen Abschnitt aufweist, als Spritzgussteil aus Kunststoff ausgebildet sein. Das Dichtelement kann nach dem Spritzgießen als separates Bauteil eingesetzt sein. Es ist jedoch auch denkbar, das elastische Dichtelement direkt an die übrige Struktur des Originalitätsverschlusses anzugießen, beispielsweise im Zweikomponentenspritzguss.

Gemäß einer weiteren möglichen Ausgestaltung kann das Dichtelement zumindest eine Aussparung aufweisen, in welche zumindest ein Vorsprung im Inneren des ersten ringförmigen Abschnittes eingreift. So kann eine formschlüssige Verbindung zwischen dem Dichtelement und dem ersten ringförmigen Abschnitt geschaffen werden. Alternativ oder zusätzlich kann das Dichtelement zumindest einen Vorsprung aufweisen, welcher in zumindest einer Ausnehmung im Inneren des ersten ringförmigen Abschnittes eingreift. Auch so kann ein formschlüssiger Eingriff zwischen Dichtelement und dem ersten ringförmigen Abschnitt geschaffen werden. Der formschlüssige Eingriff gewährleistet insbesondere eine Sicherung in axialer Richtung.

Das Dichtelement kann gemäß einer weiteren Ausführungsform zumindest eine umfängliche Nut aufweisen, welche sich vorzugsweise konzentrisch zur Längsachse des Originalitätsverschlusses erstreckt. In diese ringförmige Nut greift vorzugsweise ein ringförmiger Vorsprung im Inneren des ersten ringförmigen Abschnittes formschlüssig ein. Alternativ oder zusätzlich kann das Dichtelement zumindest einen ringförmigen Vorsprung aufweisen, welcher in zumindest eine umfängliche Nut im Inneren des ersten ringförmigen Abschnittes eingreift. Die Ausgestaltung der Vorsprünge als ringförmige Vorsprünge und die Ausgestaltung der Ausnehmung als ringförmige Nut ermöglichen eine rotationssymmetrische Form des Dichtelementes, die es ermöglicht, das Dichtelement in den ersten ringförmigen Abschnitt einzusetzen, ohne auf eine definierte Winkellage achten zu müssen.

Gemäß einer weiteren Ausgestaltung der Erfindung weist das Dichtelement einen hülsenförmigen Abschnitt auf, welcher mit seinem Innenumfang am Außenumfang des Luer-Konus anliegt. Dabei wird der hülsenförmige Anschnitt weiter bevorzugt elastisch in radialer Richtung aufgeweitet oder ausgelenkt, sodass eine Anlagekraft erzeugt wird, welche auf die Außenfläche des Luer-Konus gerichtet ist und so für eine umfängliche dichtende Anlage zwischen Dichtelement und Umfangsfläche des Luer-Konus sorgt. So wird der Luer-Konus durch das Dichtelement dicht verschlossen.

Der hülsenförmige Abschnitt des Dichtelementes kann mit seinem Außenumfang weiter bevorzugt zumindest abschnittsweise am Innenumfang des ersten ringförmigen Abschnittes anliegen. So wird eine kraftschlüssige Anlage zwischen dem Dichtelement und dem ersten ringförmigen Abschnitt geschaffen, welche eine Kraftübertragung von dem ersten ringförmigen Abschnitt auf das Dichtelement ermöglicht, um das Dichtelement gemeinsam mit dem Originalitätsverschluss von dem Luer-Konus abnehmen zu können. Ferner wird durch die Anlage zwischen Dichtelement und erstem ringförmigen Abschnitt eine gute Positionierung bzw. Führung beim Aufsetzen des Dichtelementes auf den Luer-Konus erreicht.

Vorzugsweise ist das Dichtelement in die Struktur des Originalitätsverschlusses eingesetzt bzw. mit der Struktur verbunden, bevor der Originalitätsverschluss auf die Spritze aufgesetzt wird. Zum Aufsetzen wird der Originalitätsverschluss mit dem an dem ersten ringförmigen Abschnitt ausgebildeten Außengewinde in das Innengewinde an dem äußeren zylindrischen Abschnitt des Luer-Lock-Anschluss eingeschraubt. Über den Gewindeeingriff wird bei der Drehung eine Kraft in axialer Richtung erzeugt, welche das Dichtelement auf den Luer-Konus drückt bzw. auf den Luer-Konus aufschiebt. Dies ermöglicht ein sicheres und festes Aufsetzen des Dichtelementes auf den Luer-Konus. Nach dem Aufsetzen des Originalitätsverschlusses kann dieser mit dem Spritzenzylinder verbunden werden, wobei die Verbindung vorzugsweise im Bereich der auszubildenden Sollbruchstelle oder Sollbruchstellen erfolgt.

Das Dichtelement kann weiter bevorzugt eine sich quer zur Stirnseite des Luer-Konus erstreckende Bodenfläche aufweisen, welche vorzugsweise konvex geformt ist. Diese Bodenfläche kann mit der Stirnseite des Luer-Konus in dichte Anlage treten.

Gemäß einer weiteren möglichen Ausgestaltung der Erfindung begrenzt der erste ringförmige Abschnitt des Originalitätsverschlusses eine sich bis zum zweiten, d.h. dem distalen Axialende des Originalitätsverschlusses erstreckende Ausnehmung, in welche das Dichtelement eingesetzt ist. Dies ermöglicht es, das Dichtelement vom zweiten Axialende her in die Struktur des Originalitätsverschlusses, d.h. in das Innere des ersten ringförmigen Abschnittes einzusetzen bzw. hineinzudrücken. So kann der Originalitätsverschluss sehr einfach aus zwei Teilen zusammengesetzt werden, aus einer Struktur, welche den ersten und den zweiten ringförmigen Abschnitt beinhaltet, und dem elastischen Dichtelement. Die Struktur, welche die beiden ringförmigen Abschnitte umfasst, ist vorzugsweise einstückig ausgebildet, beispielsweise als Kunststoff-Spritzgussteil.

Der erste und/oder der zweite ringförmige Abschnitt sind gemäß der Erfindung an der zumindest einen Sollbruchstelle, weiter bevorzugt an mehreren über den Umfang verteilten Sollbruchstellen mit der Stirnwand des Spritzenzylinders verschweißt. Das Verschweißen kann beispielsweise durch Ultraschalschweißen nach dem Aufsetzten des Originalitätsverschlusses auf den Luer-Lock-Anschluss erfolgen. So geschaffene Sollbruchstellen können durch Drehen des Originalitätsverschlusses gelöst werden und es lässt sich danach erkennen, dass die Sollbruchstellen getrennt bzw. zerbrochen wurden.

Zumindest eine die Sollbruchstelle aufweisende Struktur des Originalitätsverschlusses und der Spritzenzylinder sind jeweils aus Kunststoff gefertigt. Besonders bevorzugt ist, wie vorangehend beschrieben wurde, die gesamte Struktur des Originalitätsverschlusses aus Kunststoff gefertigt. Die aus Kunststoff ausgebildete Struktur umfasst insbesondere den ersten ringförmigen Abschnitt und/oder den zweiten ringförmigen Abschnitt. Dabei werden bevorzugt der die Sollbruchstelle tragende bzw. aufweisende ringförmige Abschnitt und der Spritzenzylinder aus gleichartigen Kunststoffen gefertigt, welche ein Verschweißen ermöglichen.

Alternativ zum Verschweißen des ersten und/oder des zweiten ringförmigen Abschnittes mit dem Spritzenzylinder an der Sollbruchstelle oder den Sollbruchstellen, könnten die Sollbruchstellen auch in anderer Weise, z.B. durch Verkleben ausgebildet werden. Gemäß einer weiteren möglichen Ausgestaltung könnten der Originalitätsverschluss und der Spritzenzylinder mit dem Luer-Lock-Anschluss auch durch ein Spritzgussverfahren miteinander verbunden werden, bei welchem gleichzeitig die gewünschten Sollbruchstellen ausgebildet werden.

Die Zahnflankenbreite bzw. der Querschnitt des Außengewindes an dem ersten ringförmigen Abschnitt ist vorzugsweise kleiner als die Breite des Gewindeganges am Innengewinde des äußeren zylindrischen Abschnittes des Luer-Lock-Anschlusses. So hat der Gewindeeingriff zwischen dem Außengewinde und dem Innengewinde ein axiales Spiel. Dies ermöglicht es, dass sich der Originalitätsverschluss in axialer Richtung in dem Gewindeeingriff bewegt. So kommen beim Aufsetzen des Originalitätsverschlusses auf den Luer-Lock-Anschluss die Gewindeflanken des Originalitätsverschlusses zunächst an den proximalen Gewindeflanken des Luer-Lock-Anschlusses, d.h. den der Stirnwand zugewandten Gewindeflanken, zur Anlage. Während des Verschweißens mit dem Spritzenzylinder kann der gesamte Originalitätsverschluss proximalwärts, d.h. auf die Stirnwand des Spritzenzylinders zubewegt werden, wodurch die zunächst anliegenden Gewindeflanken zuerst freigestellt werden und dann schließlich in Anlage mit den distalen Gewindeflanken des Luer-Lock-Anschlusses bewegt werden. Die distalen Gewindeflanken des Luer-Lock-Anschlusses sind der Stirnwand abgewandt.

In das erste Axialende, d.h. das proximale Axialende des Spritzenzylinders ist weiter bevorzugt ein axial verschiebbarer Stopfen eingesetzt. Dieser axial verschiebbare Stopfen dient als Kolben der Spritze und verschließt gleichzeitig das erste Axialende.

Besonders bevorzugt kann die Spritze einen vorgefüllten Spritzenzylinder aufweisen. D.h. die Spritze ist mit einem pharmazeutischen Produkt vorgefüllt und wird so an den Kunden ausgeliefert. Dieser öffnet dann den Originalitätsverschluss und kann die Spritze durch Verschieben des Stopfens bzw. Kolbens in der Axialrichtung hindurch entleeren.

Der Spritzenzylinder kann beispielsweise durch den Luer-Konus vorgefüllt werden, wobei sich der Kolben oder Stopfen in dem Spritzenzylinder zu dem ersten Axialende bewegt. Danach kann der Originalitätsverschluss mit dem Dichtelement auf den Luer-Lock-Anschluss aufgesetzt werden und anschließend verschweißt werden.

Nachfolgend wird die Erfindung beispielhaft anhand der beigefügten Figuren beschrieben. In diesen zeigt:
- Figur 1: eine Schnitteinsicht eines distalen Endes einer Spritze mit einem aufgesetzten Originalitätsverschluss,
- Figur 2: eine Seitenansicht eines Originalitätsverschlusses,
- Figur 3: eine Schnittansicht des Originalitätsverschlusses gemäß Figur 2 ohne Dichtelement,

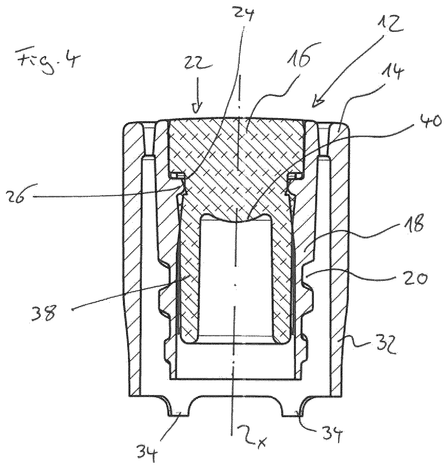

- Figur 4: eine Schnittansicht entsprechend Figur 3 mit eingesetztem Dichtelement,
- Figur 5: einen Originalitätsverschluss gemäß einer zweiten Ausführungsform der Erfindung, und
- Figur 6: eine Schnittansicht des Originalitätsverschlusses gemäß Figur 5.

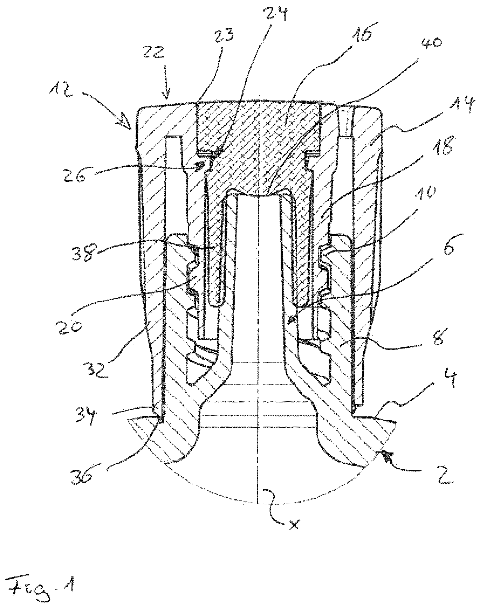

Figur 1 zeigt in einer Schnittansicht das distale Ende einer Spritze bzw. eines Spritzenzylinders 2. An seinem ersten, d.h. dem proximalen Ende ist der Spritzenzylinder in üblicher Weise offen ausgebildet und durch einen Kolben bzw. Stopfen verschlossen, welcher hier nicht gezeigt ist. An dem gezeigten zweiten Axialende, gesehen in Richtung der Längsachse X, welches das distale Ende darstellt, weist der Spritzenzylinder 2 eine Stirnwand 4 auf, von welcher ausgehend sich in distaler Richtung ein Luer-Lock-Anschluss erstreckt. Der Luer-Lock-Anschluss weist in üblicher Weise im Inneren einen Luer-Konus 6 auf, welcher radial beabstandet von einem äußeren zylindrischen Abschnitt 8 umgeben ist, welcher an seinem Innenumfang ein Innengewinde 10 aufweist. Der Luer-Lock-Anschluss mit dem Luer-Konus 6 und dem äußeren zylindrischen Abschnitt 8 mit dem Innengewinde 10 ist in üblicher Weise, insbesondere normgerecht, ausgestaltet.

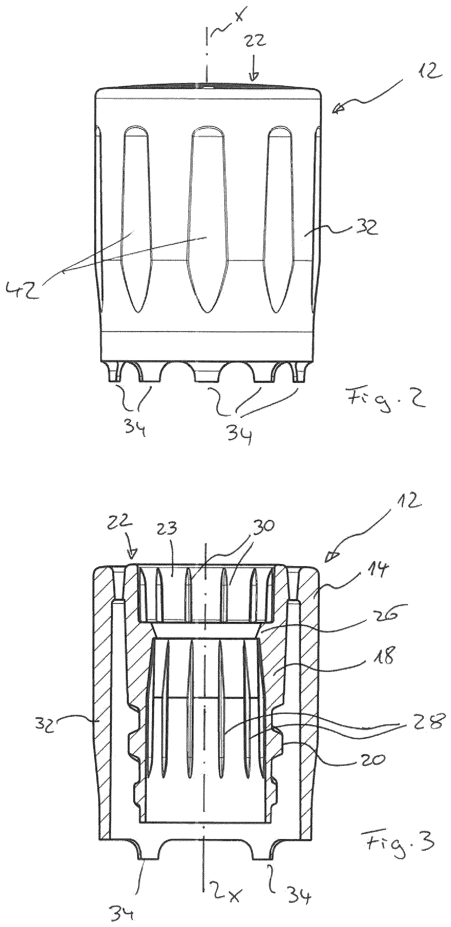

Die gezeigte Spritze ist durch einen Originalitätsverschluss 12 verschollen bzw. versiegelt. Der Originalitätsverschluss 12 ist aus einer einstückigen Kunststoffstruktur 14 gebildet, in welche ein elastisches Dichtelement 16 eingesetzt ist. Die Kunststoffstruktur 14 weist einen ersten ringförmigen Abschnitt 18 auf, welcher an seinem Außenumfang ein Außengewinde 20 hat. Der erste ringförmige Abschnitt 18 mit dem Außengewinde 20 ist so dimensioniert, dass der erste ringförmige Abschnitt 18 in den äußeren zylindrischen Abschnitt des Luer-Lock-Anschlusses eingreifen kann, wobei das Außengewinde 20 in das Innengewinde 10 schraubend eingreift. Der erste ringförmige Abschnitt 18 bildet einen Zylinder, welcher den Luer-Konus 6 beabstandet umgibt. Der erste ringförmige Abschnitt 18 definiert einen rohrförmigen Innenraum, welcher sich bis zum axialen Ende 22 des Originalitätsverschlusses 12 erstreckt und in einer Öffnung 23 in der Stirnseite endet. In diese zentrale Öffnung 23 ist das Dichtelement 16, welches aus einem Elastomermaterial, beispielsweise Gummi gefertigt ist, eingesetzt. Das Dichtelement 16 wird dabei kraft- und formschlüssig im Inneren der Öffnung und des ersten ringförmigen Abschnittes 18 gehalten. Das Dichtelement 16 ist rotationssymmetrisch zur Längsachse X ausgebildet und weist eine umfängliche Nut 24 auf, in welche ein umfänglicher Vorsprung 26, welcher am Innenumfang des ersten ringförmigen Abschnittes 18 auskragt, eingreift. So wird eine formschlüssige Fixierung in axialer Richtung X erreicht. Darüber hinaus weist, um eine gute Kraftübertragung im Umfangsrichtung erreichen zu können, der erste ringförmige Abschnitt 18, der in Figur 3 zu erkennen ist, über den Umfang verteilt, rippenförmige Vorsprünge 28 auf. Diese rippenförmige Vorsprünge 28 erstrecken sich im Wesentlichen parallel zur Längsachse X. Auch in der an das Axialende angrenzenden Öffnung 23 sind über den Umfang verteilt vorstehende Rippen 30 angeformt. Die rippenförmige Vorsprünge 28 und die Rippen 30 greifen formschlüssig in das Material des Dichtelementes 16 ein, wobei das Dichtelement 16 verformt wird. So wird ein kraft- und formschlüssiger Eingriff erreicht, welcher eine Kraftübertragung in Umfangsrichtung ermöglicht. Bei Drehung des Originalitätsverschlusses 12 in dem Gewinde 10 wird das Dichtelement 16 so sicher mitgedreht.

In diesem ersten Ausführungsbeispiel weist der Originalitätsverschluss 12 ferner einen zweiten ringförmigen Abschnitt 32 auf, welcher die äußere Umfangsfläche des Originalitätsverschlusses 12 bildet. Der zweite ringförmige Abschnitt erstreckt sich hülsenförmig ausgehend vom distalen bzw. zweiten Axialende 22 zu der Stirnwand 4 des Spritzenzylinders 2. Der zweite ringförmige Abschnitt 32 ist in radialer Richtung von dem ersten inneren ringförmigen Abschnitt 18 beabstandet, sodass zwischen beiden ein Freiraum ausgebildet ist, in welchen der äußere zylindrische Abschnitt 8 des Luer-Lock-Anschlusses eingreift.

In diesem ersten Ausführungsbeispiel weist der zweite ringförmige Abschnitt 32 an seinem ersten Axialende über den Umfang verteilt mehrere axial gerichtete Vorsprünge 34 auf, welche die Stirnwand 4 des Spritzenzylinders 2 berühren und mit dieser verschweißt sind. Über die Vorsprünge 34 und eine Schweißnaht 36 wird eine als Sollbruchstelle ausgebildete Verbindung zu der Stirnwand 4 des Spritzenzylinders 2 geschaffen. Wenn der so, beispielsweise durch Ultraschalschweißen, mit dem Spritzenzylinder verbundene Originalitätsverschluss 12 gedreht wird, reißen die Vorsprünge 34 an den Schweißnähten 36 von der Stirnwand 4 ab, d.h. die Sollbruchstellen werden aufgerissen bzw. aufgebrochen und der Originalitätsverschluss 4 kann in den Innengewinde 10 des Luer-Lock-Anschlusses gedreht werden und von dem Luer-Konus 6 abgenommen werden.

Zur Abdichtung weist das Dichtelement 16 einen hülsenförmigen Abschnitt 38 auf, welcher sich in axialer Richtung X proximalwärts zu dem Spritzenzylinder 2 hin erstreckt. Der hülsenförmige Abschnitt 38 ist an seiner dem Spritzenzylinder 2 zugewandten Seite geöffnet und nimmt in diesem Bereich das distale Ende des Luer-Konus 6 auf. Dabei wird der hülsenförmige Abschnitt 38 elastisch aufgeweitet und kommt auf der Umfangsfläche des Luer-Konus 6 dichtend zu liegen. An seinem distalen Ende ist der hülsenförmige Abschnitt 38 durch eine Bodenfläche 40 des Dichtelementes 16 verschlossen. Die Bodenfläche 40 ist konkav gewölbt und kommt ggf. zusätzlich dichtend an der Stirnseite des Luer-Konus 6 zur Anlage.

Wie in der Figur 1 zu erkennen ist, weist der Eingriff zwischen dem Außengewinde 20 und dem Innengewinde 10 ein axiales Spiel auf, d.h. die Gewindevorsprünge des Außengewindes 20 sind in axialer Richtung X schmaler ausgebildet als die Gewindenuten bzw. Gewindegänge des Innengewindes 10. Dies ermöglicht es, den Originalitätsverschluss 12 bzw. dessen Kunststoffstruktur 14 während des Verschweißens mit der Stirnwand 4 in axialer Richtung zu dem Spritzenzylinder 2 hin zu verschieben. Beispielsweise kann das Außengewinde 20 zunächst an den proximalwärts gewandten Gewindeflanken des Innengewindes 20 anliegen und dann nach dem axialen Verschieben an den distalseitigen Gewindeflanken des Innengewindes anliegen.

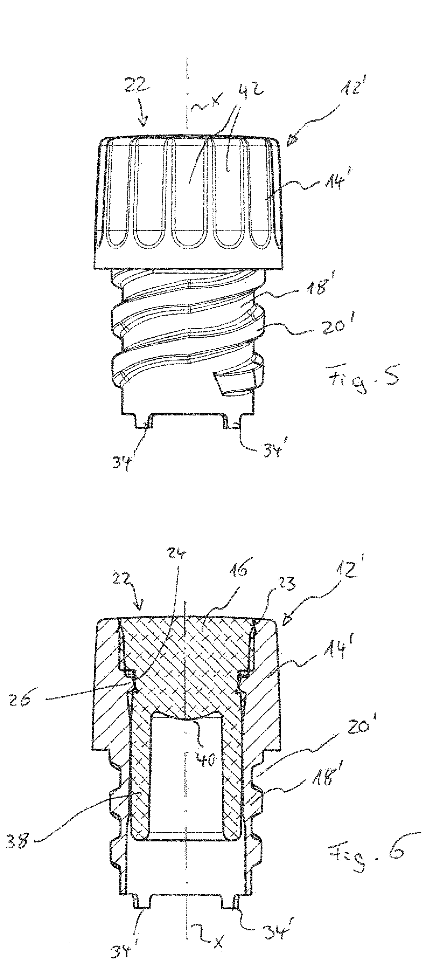

Die Figuren 5 und 6 zeigen ein zweites Ausführungsbeispiel eines erfindungsgemäßen Originalitätsverschlusses, welches sich von dem ersten Ausführungsbeispiel dahingehend unterscheidet, dass es keinen zweiten ringförmigen Abschnitt gibt. In diesem Ausführungsbeispiel weist der Originalitätsverschluss 12' in seiner Kunststoffstruktur 14' lediglich einen ringförmigen Abschnitt 18' auf, welcher Außengewinde 20' trägt, welches entsprechend dem Außengewinde 20, wie es bei dem ersten Ausführungsbeispiel beschrieben wurde, ausgebildet ist. Das Außengewinde 20' tritt auch in diesem Ausführungsbeispiel mit dem Innengewinde 10 des Luer-Lock-Anschlusses in Eingriff. Am proximalen, d.h. ersten Axialende, weist der ringförmige Abschnitt 38' axial gerichtete Vorsprünge 34' auf, die über Schweißverbindungen mit dem Spritzenzylinder 2 verbunden werden können, wobei die Schweißverbindungen Sollbruchstellen bilden. Die Verbindung mit dem Spritzenzylinder erfolgt bei diesem Ausführungsbeispiel somit in dem Freiraum zwischen dem Luer-Konus 6 und dem äußeren zylindrischen Abschnitt 8 des Luer-Lock-Verbinders, d.h. am Boden dieses ringförmigen Freiraums. Diese Verbindung kann durch Drehen gelöst bzw. abgerissen werden, sodass auch hier eine nicht reversible Verbindung zwischen dem Originalitätsverschluss 12' und dem Spritzenzylinder 2 geschaffen wird, anhand derer zu erkennen ist, ob der Originalitätsverschluss 12' zuvor geöffnet wurde oder nicht. Dies lässt sich besonders gut erkennen, wenn der Spritzenzylinder 2 mit dem Luer-Lock-Anschluss und auch die Kunststoffstruktur 14, 14' aus transparentem Kunststoff ausgebildet sind.

Das Dichtelement 16 ist bei dem zweiten Ausführungsbeispiel entsprechend dem vorangehend beschriebenen ersten Ausführungsbeispiel ausgestaltet. Auch der Eingriff des Außengewindes 20' mit dem Innengewinde 10 mit axialem Spiel kann in derselben Weise realisiert sein, wie es oben beschrieben wurde. In beiden Ausführungsbeispielen ist der Außenumfang des Originalitätsverschlusses 12 mit Riffelungen 42 versehen, um eine gute Kraftübertragung beim Drehen zu ermöglichen.

### Bezugszeichenliste

- 2: Spritzenzylinder
- 4: Stirnwand
- 6: Luer-Konus
- 8: äußerer zylindrischer Abschnitt
- 10: Innengewinde
- 12, 12': Originalitätsverschluss
- 14, 14': Kunststoffstruktur
- 16: Dichtelement
- 18, 18': erster ringförmiger Abschnitt
- 20, 20': Außengewinde
- 22: axiales Ende
- 23: Öffnung
- 24: umfängliche Nut
- 26: umfänglicher Vorsprung
- 28: rippenförmige Vorsprünge
- 30: Rippen
- 32: zweiter ringförmiger Vorsprung
- 34, 34: axiale Vorsprünge
- 36: Schweißnaht
- 38: hülsenförmiger Abschnitt
- 40: Bodenfläche
- 42: Riffelungen
- X: Längsachse

## Patentansprüche

1. Spritze mit einem Spritzenzylinder (2), der an einem ersten Axialende offen ausgebildet ist und an seinem entgegengesetzten zweiten Axialende eine Stirnwand (4) mit einem an dieser ausgebildeten Luer-Lock-Anschluss (6, 8) aufweist, sowie einem den Luer-Lock-Anschluss (6, 8) verschließenden und mit dem Spritzenzylinder (2) über zumindest eine Sollbruchstelle (36) verbundenen Originalitätsverschluss (12, 12'), wobei
der Originalitätsverschluss (12, 12') einen ersten ringförmigen Abschnitt (18, 18') aufweist, der mit einem Außengewinde (20, 20') versehen ist, welches in das Innengewinde (10) am äußeren zylindrischen Abschnitt (8) des Luer-Lock-Anschlusses eingreift
**dadurch gekennzeichnet, dass**
der erste ringförmige Abschnitt (18') an einem ersten Axialende über die zumindest eine Sollbruchstelle (34') mit dem Spritzenzylinder (2) verbunden ist, indem der erste ringförmige Abschnitt (18') an der zumindest einen Sollbruchstelle (34') mit der Stirnwand (4) des Spritzenzylinders (2) verschweißt ist, und/oder dass der Originalitätsverschluss (12) mit einem zweiten ringförmigen Abschnitt (32) versehen ist, welcher den äußeren zylindrischen Abschnitt (8) des Luer-Lock-Anschlusses umfänglich umgibt, und der zweite ringförmige Abschnitt (32) an einem ersten Axialende über die zumindest eine Sollbruchstelle (34, 36) mit dem Spritzenzylinder (2) verbunden ist, indem der zweite ringförmige Abschnitt (32) an derzumindest einen Sollbruchstelle (34, 36) mit der Stirnwand (4) des Spritzenzylinders (2) verschweißt ist.

2. Spritze nach Anspruch 1, **dadurch gekennzeichnet, dass** der Originalitätsverschluss (12) ein an dem Luerkonus (6) des Luer-Lock-Anschlusses anliegendes elastisches Dichtelement (16) aufweist, welches vorzugsweise im Inneren des ersten ringförmigen Abschnittes (18, 18') angeordnet ist.

3. Spritze nach Anspruch 2, **dadurch gekennzeichnet, dass** das Dichtelement (16) kraft- und/oder formschlüssig in dem ersten ringförmigen Abschnitt (18, 18') fixiert ist, wobei vorzugsweise das Dichtelement (16) zumindest eine Aussparung (2) aufweist, in welche zumindest ein Vorsprung (36) im Inneren des ersten ringförmigen Abschnittes (18, 18') eingreift, und/oder das Dichtelement (16) zumindest einen Vorsprung aufweist, welcher in zumindest eine Ausnehmung im Inneren des ersten ringförmigen Abschnittes eingreift.

4. Spritze nach Anspruch 2 oder 3, **dadurch gekennzeichnet, dass** das Dichtelement (16) zumindest eine umfängliche Nut (24) aufweist, in welche zumindest ein ringförmiger Vorsprung (26) im Inneren des ersten ringförmigen Abschnittes (18, 18') eingreift, und/oder dass das Dichtelement (16) zumindest einen ringförmigen Vorsprung aufweist, welcher in zumindest eine umfängliche Nut im Inneren des ersten ringförmigen Abschnittes eingreift.

5. Spritze nach einem der Ansprüche 2 bis 4 **dadurch gekennzeichnet, dass** das Dichtelement (16) einen hülsenförmigen Abschnitt (38) aufweist, welcher mit seinem Innenumfang am Außenumfang des Luerkonus (6) anliegt.

6. Spritze nach Anspruch 5, **dadurch gekennzeichnet, dass** der hülsenförmige Abschnitt (38) mit seinem Außenumfang zumindest abschnittweise am Innenumfang des ersten ringförmigen Abschnittes (18, 18') anliegt.

7. Spritze nach einem der Ansprüche 2 bis 6, **dadurch gekennzeichnet, dass** das Dichtelement (16) eine sich quer zur Stirnseite des Luerkonus (6) erstreckende Bodenfläche (40) aufweist, welche vorzugsweise konvex geformt ist.

8. Spritze nach einem der Ansprüche 2 bis 7, **dadurch gekennzeichnet, dass** der erste ringförmige Abschnitt (18, 18') des Originalitätsverschlusses (12) eine sich bis zum zweiten Axialende des Originalitätsverschlusses (12) erstreckende Ausnehmung begrenzt, in welche das Dichtelement (16) eingesetzt ist.

9. Spritze nach einem der vorangehenden Ansprüche, **dadurch gekennzeichnet, dass** der erste (18, 18') und/oder zweite (32) ringförmige Abschnitt an mehreren über den Umfang verteilte Sollbruchstellen (34, 34', 36) mit der Stirnwand (4) des Spritzenzylinders (2) verschweißt sind.

10. Spritze nach einem der vorangehenden Ansprüche, **dadurch gekennzeichnet, dass** zumindest eine die Sollbruchstelle aufweisende Struktur (14) des Originalitätsverschlusses und der Spritzenzylinder (2) jeweils aus Kunststoff und bevorzugt aus gleichartigem Kunststoff gefertigt sind.

11. Spritze nach einem der vorangehenden Ansprüche, **dadurch gekennzeichnet, dass** die Zahnflankenbreite des Außengewindes (20, 20') an dem ersten ringförmigen Abschnitt (18, 18') kleiner ist als die Breite des Gewindeganges des Innengewindes (10) am äußeren zylindrischen Abschnitt (8) des Luer-Lock-Anschlusses.

12. Spritze nach einem der vorangehenden Ansprüche, **dadurch gekennzeichnet, dass** in das erste Axialende des Spritzenzylinders (2) ein axial verschiebbarer Stopfen eingesetzt ist.

13. Spritze nach einem der vorangehenden Ansprüche, **dadurch gekennzeichnet, dass** der Spritzenzylinder (2) vorgefüllt ist.

## Claims

1. A syringe with a syringe cylinder (2), which is configured in an open manner at a first axial end, and at its opposite second axial end has a front wall (4) with a Luer lock connection (6, 8) formed thereon, and with a tamper-proof closure (12, 12') closing the Luer lock connection (6, 8) and connected to the syringe cylinder (2) via at least one predetermined breaking point (36), wherein
the tamper-proof closure (12, 12') has a first ring-shaped portion (18, 18') which is provided with an external thread (20, 20') which engages into the internal thread (10) on the outer cylindrical portion (8) of the Luer lock connection,
**characterized in that**
the first ring-shaped portion (18') is connected to the syringe cylinder (2) at a first axial end via the at least one predetermined breaking point (34'), by the first ring-shaped portion (18') at the at least one predetermined breaking point (34') being welded to the front wall (4) of the syringe cylinder (2), and/or that
the tamper-proof closure (12) is provided with a second ring-shaped portion (32), which surrounds the outer cylindrical portion (8) of the Luer lock connection circumferentially, and the second ring-shaped portion (32) at a first axial end is connected to the syringe cylinder (2) via the at least one predetermined breaking point (34, 36), by the second ring-shaped portion (32) at the at least one predetermined breaking point (34, 36) being welded to the front wall (4) of the syringe cylinder (2).

2. The syringe according to Claim 1, **characterized in that** the tamper-proof closure (12) has an elastic sealing element (16) lying against the Luer cone (6) of the Luer lock connection, which is preferably arranged in the interior of the first ring-shaped portion (18, 18').

3. The syringe according to Claim 2, **characterized in that** the sealing element (16) is fixed in a force-fitting and/or form-fitting manner in the first ring-shaped portion (18, 18'), wherein preferably the sealing element (16) has at least one recess (2), into which at least one projection (36) engages in the interior of the first ring-shaped portion (18, 18'), and/or the sealing element (16) has at least one projection, which engages into at least one opening in the interior of the first ring-shaped portion.

4. The syringe according to Claim 2 or 3, **characterized in that** the sealing element (16) has at least one circumferential groove (24), into which at least one ring-shaped projection (26) engages in the interior of the first ring-shaped portion (18, 18'), and/or that the sealing element (16) has at least one ring-shaped projection, which engages into at least one circumferential groove in the interior of the first ring-shaped portion.

5. The syringe according to one of Claims 2 to 4, **characterized in that** the sealing element (16) has a sleeve-shaped portion (38), which lies with its inner circumference against the outer circumference of the Luer cone (6).

6. The syringe according to Claim 5, **characterized in that** the sleeve-shaped portion (38) lies with its outer circumference at least partially against the inner circumference of the first ring-shaped portion (18, 18').

7. The syringe according to one of Claims 2 to 6, **characterized in that** the sealing element (16) has a base surface (40) extending transversely to the front face of the Luer cone (6), which is preferably shaped in a convex manner.

8. The syringe according to one of Claims 2 to 7, **characterized in that** the first ring-shaped portion (18, 18') of the tamper-proof closure (12) delimits an opening extending up to the second axial end of the tamper-proof closure (12), into which the sealing element (16) is inserted.

9. The syringe according to one of the preceding claims, **characterized in that** the first (18, 18') and/or second (32) ring-shaped portion are welded to the front wall (4) of the syringe cylinder (2) at several predetermined breaking points (34, 34', 36) distributed over the circumference.

10. The syringe according to one of the preceding claims, **characterized in that** at least one structure (14) of the tamper-proof closure having the predetermined breaking point, and the syringe cylinder (2) are manufactured respectively from plastic and preferably from the same type of plastic.

11. The syringe according to one of the preceding claims, **characterized in that** the tooth flank width of the external thread (20, 20') on the first ring-shaped portion (18, 18') is smaller than the width of the thread pitch of the internal thread (10) on the outer cylindrical portion (8) of the Luer lock connection.

12. The syringe according to one of the preceding claims, **characterized in that** an axially displaceable stopper is inserted into the first axial end of the syringe cylinder (2).

13. The syringe according to one of the preceding claims, **characterized in that** the syringe cylinder (2) is pre-filled.

## Revendications

1. Seringue, dotée d'un cylindre de seringue (2), qui sur une première extrémité axiale est conçu en étant ouvert et sur sa deuxième extrémité axiale opposée, comporte une paroi frontale (4), dotée d'un raccord Luer-Lock (6, 8) conçue sur celle-ci et d'une fermeture inviolable (12, 12'), la fermeture inviolable (12, 12') fermant le raccord Luer-Lock(6, 8) et assemblée avec le cylindre de seringue (2) par l'intermédiaire d'au moins une zone de rupture théorique (36),
la fermeture inviolable (12, 12') comportant un premier segment (18, 18') de forme annulaire, qui est muni d'un filetage (20, 20'), lequel s'engage dans le taraudage (10) sur le segment (8) cylindrique extérieur du raccord Luer-Lock,
**caractérisée en ce que**
sur une première extrémité axiale, le premier segment (18') de forme annulaire est assemblé par l'intermédiaire de l'au moins une zone de rupture théorique (34') avec le cylindre de seringue (2), **en ce que** le premier segment (18') de forme annulaire de l'au moins une zone de rupture théorique (34') est soudé avec la paroi frontale (4) du cylindre de seringue (2) et / ou **en ce que**
la fermeture inviolable (12) est munie d'un deuxième segment (32) de forme annulaire, lequel entoure sur sa périphérie le segment (8) cylindrique externe du raccord Luer-Lock et sur une première extrémité axiale, le deuxième segment (32) de forme annulaire est assemblé par l'intermédiaire de l'au moins une zone de rupture théorique (34, 36) avec le cylindre de seringue (2), **en ce que** le deuxième segment (32) de forme annulaire est soudé sur l'au moins une zone de rupture théorique (34, 36) avec la paroi frontale (4) du cylindre de seringue (2).

2. Seringue selon la revendication 1, **caractérisée en ce que** la fermeture inviolable (12) comporte un élément d'étanchéité (16) élastique, adjacent au cône Luer (6) du raccord Luer-Lock, lequel est placé de préférence à l'intérieur du premier segment (18, 18') de forme annulaire.

3. Seringue selon la revendication 2, **caractérisée en ce que** l'élément d'étanchéité (16) est fixé par complémentarité de force et / ou de forme dans le premier segment (18, 18') de forme annulaire, de préférence, l'élément d'étanchéité (16) comportant au moins une encoche (2), dans laquelle s'engage au moins une saillie (36) à l'intérieur du premier segment (18, 18') de forme annulaire et / ou **en ce que** l'élément d'étanchéité (16) comporte au moins une saillie, laquelle s'engage dans au moins un évidement, à l'intérieur du premier segment de forme annulaire.

4. Seringue selon la revendication 2 ou 3, **caractérisée en ce que** l'élément d'étanchéité (16) comporte au moins une rainure (24) périphérique, dans laquelle au moins une saillie (26) de forme annulaire s'engage à l'intérieur du premier segment (18, 18') de forme annulaire et / ou **en ce que** l'élément d'étanchéité (16) comporte au moins une saillie de forme annulaire, laquelle s'engage dans au moins une rainure périphérique, à l'intérieur du premier segment de forme annulaire.

5. Seringue selon l'une quelconque des revendications 2 à 4, **caractérisée en ce que** l'élément d'étanchéité (16) comporte un segment (38) en forme de douille, lequel par sa périphérie intérieure est adjacent à la périphérie extérieure du cône Luer (6).

6. Seringue selon la revendication 5, **caractérisée en ce que** par sa périphérie extérieure, le segment (38) en forme de douille est adjacent au moins par endroits à la périphérie intérieure du premier segment (18, 18') de forme annulaire.

7. Seringue selon l'une quelconque des revendications 2 à 6, **caractérisée en ce que** l'élément d'étanchéité (16) comporte une surface de fond inférieur (40) s'étendant à la transversale de la face frontale du cône Luer (6), laquelle est façonnée de préférence de forme convexe.

8. Seringue selon l'une quelconque des revendications 2 à 7, **caractérisée en ce que** le premier segment (18, 18') de forme annulaire de la fermeture inviolable (12) délimite un évidement s'étendant jusqu'à la deuxième extrémité axiale de la fermeture inviolable (12), dans lequel est inséré l'élément d'étanchéité (16).

9. Seringue selon l'une quelconque des revendications précédentes, **caractérisée en ce que** sur plusieurs zones de rupture théorique (34, 34', 36) distribuées sur la périphérie, le premier (18, 18') et / ou le deuxième (32) segment de forme annulaire est soudé avec la paroi frontale (4) du cylindre de seringue (2).

10. Seringue selon l'une quelconque des revendications précédentes, **caractérisée en ce qu'**au moins une structure (14) de la fermeture inviolable comportant la zone de rupture théorique et le cylindre de seringue (2) sont fabriqués chacun en une matière plastique et de manière préférentielle, en une matière plastique de même type.

11. Seringue selon l'une quelconque des revendications précédentes, **caractérisée en ce que** la largeur de flanc de dent du filetage (20, 20') sur le premier segment (18, 18') de forme annulaire est inférieure à la largeur du pas de vis du taraudage (10) sur le segment (8) cylindrique extérieur du raccord Luer-Lock.

12. Seringue selon l'une quelconque des revendications précédentes, **caractérisée en ce que** dans la première extrémité axiale du cylindre de seringue (2) est inséré un bouchon déplaçable en direction axiale.

13. Seringue selon l'une quelconque des revendications précédentes, **caractérisée en ce que** le cylindre de seringue (2) est prérempli.
